(19) Europäisches Patentamt
European Patent Office
Office européen des brevets

(11) Publication number: **0 021 229**
**B1**

(12) **EUROPEAN PATENT SPECIFICATION**

(45) Date of publication of patent specification: **22.12.82**

(21) Application number: **80103217.8**

(22) Date of filing: **10.06.80**

(51) Int. Cl.[3]: **C 07 C 107/06,**
C 07 D 213/76,
C 07 D 239/69,
C 07 D 263/50,
C 07 D 405/12,
A 61 K 31/655

# (54) Arylazo compounds, their preparation and their use.

(30) Priority: **11.06.79 SE 7905051**

(43) Date of publication of application:
**07.01.81 Bulletin 81/1**

(45) Publication of the grant of the patent:
**22.12.82 Bulletin 82/51**

(84) Designated Contracting States:
**BE CH DE FR GB IT LI NL SE**

(56) References cited:
**DE - A - 2 257 629**

(73) Proprietor: **PHARMACIA AB**
**Box 181**
**S-751 04 Uppsala (SE)**

(72) Inventor: **Agback, Karl Hubert**
**Mimervägen 7**
**S-754 40 Uppsala (SE)**

(74) Representative: **Kraus, Walter, Dr. et al,**
**Patentanwälte Dres. Kraus & Weisert**
**Irmgardstrasse 15**
**D-8000 München 71 (DE)**

**EP 0 021 229 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Arylazo compounds, their preparation and their use

The present invention relates to new arylazo compounds, pharmaceutical preparations containing these compounds and processes for the preparation of the compounds.

Arylazo compounds are already described in DE—A—2 257 629. As stated in this publication, these arylazo compounds are characterized by an immuno suppressive activity.

The object of the present invention is to provide new arylazo compounds having the surprisingly strong inhibiting effect on enzymes, which synthetize and inactivate prostaglandins, whereby they can influence and modify the effect of the prostaglandins.

The new compounds according to the invention are represented by the general formula

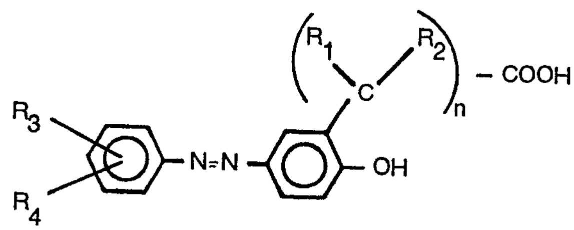

I

wherein $R_1$ and $R_2$ independently of each other represent hydrogen or $C_{1-3}$ alkyl, $R_3$ represents hydrogen, halogen, $C_{1-3}$ alkyl, $C_{1-3}$ alkoxy, carboxy, $C_{1-3}$ alkylcarbonyl, $C_{1-3}$ alkoxycarbonyl or a group $X—NH—SO_2—$, wherein X represents a heterocyclic ring, $R_4$ represents hydrogen, halogen or $C_{1-3}$ alkyl, and n is the integer 1 or 2, the two groups

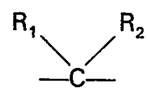

being the same or different when $n = 2$.
and lactones as well as pharmacologically acceptable salts thereof.

When the radicals $R_1—R_4$ represent or contain $C_{1-3}$ alkyl or $C_{1-3}$ alkoxy the latter groups are preferably methyl or ethyl, and methoxy or ethoxy respectively, particularly methyl and methoxy. The symbol X preferably represents a monocyclic, suitably unsaturated heterocyclic ring, which preferably has 5—6 ring members. The heterocyclic ring preferably contains one or two hetero atoms, selected from nitrogen, oxygen and sulfur. As examples of such heterocyclic ring systems pyridyl, pyrimidyl, pyrazinyl, oxazole, thiazole, and the like may be mentioned. The heterocyclic ring X may optionally be substituted by one or more substituents selected from halogen and low-alkyl (having 1—6 carbon atoms, particularly 1—3 carbon atoms). Examples of suitable pharmacologically acceptable salts are alkali metal, alkaline-earth metal and (optionally substituted) ammonium salts.

As examples of suitable compounds according to the invention the following compounds may be mentioned:

2-hydroxy-5[4-[[(2-pyridinyl)-amino]sulfonyl]phenylazo]benzeneacetic acid,
2-hydroxy-5-[4-[[(2-pyridinyl)-amino]sulfonyl]phenylazo]benzenepropanoic acid,
2-hydroxy-5-[4-[[(3,4-dimethyl-5-isoxazolyl)amino]sulfonyl]phenylazo]benzeneacetic acid,
2-hydroxy-5-[4-[[(4,5-dimethyl-2-oxazolyl)amino]sulfonyl]phenylazo]benzenepropanoic acid,
2-hydroxy-5-[4-[[(4,6-dimethyl-2-pyrimidinyl)-amino]sulfonyl]phenylazo]benzenepropanoic acid,
2-hydroxy-5-[4-[[(4,6-dimethyl-2-pyrimidinyl)-amino]sulfonyl]phenylazo]benzeneacetic acid,
2-hydroxy-5-[4-[[(2-pyridinyl)amino]-sulfonyl]phenylazo]-$\alpha$,$\alpha$-dimethyl-benzeneacetic acid,
2-hydroxy-5-phenylazo-benzeneacetic acid,
2-hydroxy-5-phenylazo-benzenepropanoic acid,
2-hydroxy-5-(4-methoxy-phenylazo)benzeneacetic acid,
5-(4-chloro-phenylazo)-2-hydroxy-benzeneacetic acid,
5-(3-chloro-phenylazo)-2-hydroxy-benzeneacetic acid,
5-(2-chloro-phenylazo)-2-hydroxy-benzenepropanoic acid,
2-hydroxy-5-(4-methyl-phenylazo)benzenepropanoic acid,
5-(4-ethoxycarbonyl-phenylazo)-2-hydroxy-benzeneacetic acid,
5-(3-chloro-4-methyl-phenylazo)-2-hydroxy-benzeneacetic acid,
5-(3-chloro-4-methyl-phenylazo)-2-hydroxy-benzenepropanoic acid,
5-(4-carboxy-phenylazo)-2-hydroxy-benzeneacetic acid,
4-[(2-oxo-5(2H)-benzofuranyl)-azo]-N-(2-pyridinyl)benzenesulfonamide,
4-[(3,4-dihydro-2-oxo-2H-1-benzopyran-6-yl)-azo]-N-(2-pyridinyl)benzenesulfonamide.

The compounds of formula I according to the invention can be prepared by several different methods known per se, e.g. according to one of the following methods:

1. Diazotization of a compound of the general formula II

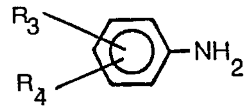

II

wherein $R_3$ and $R_4$ are as defined above, and coupling in an alkaline medium with a compound of the general formula III

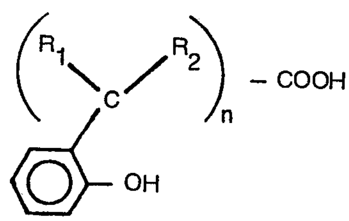

III

wherein $R_1$, $R_2$ and n are as defined above.

2. Reaction of a sulfonyl chloride of the formula

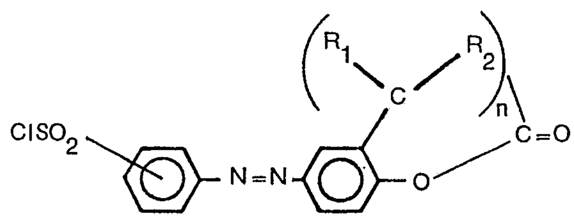

IV

wherein $R_1$, $R_2$ and n are as defined above, with an amine of the formula

$$X—NH_2 \qquad V$$

wherein X is as defined above, in the presence of a basic condensation agent.

The starting material of method 2) may e.g. be obtained from sulfanilic acid, which is diazotized and coupled with a compound of the formula III analogously with the described method 1), whereupon the azo compound formed through treatment with a dehydrating agent is converted into a lactone of the general formula

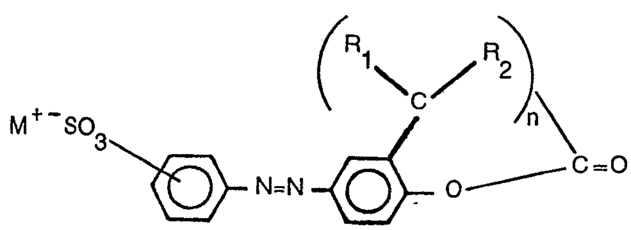

VI

wherein $R_1$, $R_2$ and n are as defined above and $M^+$ is an alkali metal cation. The starting material of formula IV is then obtained in known manner through chlorination of the sulfonate VI.

The compounds of formula I are easily converted into the corresponding lactones and vice versa. For example, the open-ring acid of formula I is converted into the corresponding lactone by heating, particularly in acidic medium. The compounds are generally soluble in organic solvents in their lactone form, while in buffered neutral or basic aqueous systems they exist as soluble salts of the open-ring form.

As mentioned above the new compounds of formula I, as well as the corresponding lactones and salts, are characterized by a surprisingly high inhibiting effect on prostaglandin synthetizing and inactivating enzymes, which has been verified in i.a. experiments relating to the effect on the prostaglandin metabolism of human placenta. They may therefore be used i.a. in treatment of inflammatory diseases, such as rheumatoid arthritis and ulcerative colitis, as male anti-fertility agents, etc. The inhibiting effect of the compounds on the inactivation of prostaglandins has been shown by experiments in vitro as follows:

Radioactive prostaglandin $F_{2\alpha}$ was incubated with cellfree preparations of different organs (containing prostaglandin-15-dehydrogenase) according to Hoult and Moore, British Journal of Pharmacology, Vol. 61 (1977), p. 615. After a suitable time the prostaglandins were extracted and separated by chromatography. Then the amount of remaining prostaglandin $F_{2\alpha}$ was compared with the amounts of metabolites. The inhibiting effect was obtained by the addition of the compounds according to the invention during the incubation and comparison with control experiments without such addition. The results may be represented either as a concentration giving 50% inhibition of the prostaglandin inactivation, or as an inhibition in per cent at a certain concentration of the studied compound. In the following table the results for some compounds according to the invention are shown compared to the known drug sulfasalazine.

In the table, compound
A is a sulfasalazine,
B is 2-hydroxy-5-[4-[[(2-pyridinyl)amino]sulfonyl]phenylazo]benzeneacetic acid,
C is 2-hydroxy-5-phenylazo-benzeneacetic acid.

TABLE

| Organ | Compound | Concentration μM | (μg/ml) | Inhibition, % |
|---|---|---|---|---|
| Human placenta | A | 95 | (38) | 50 |
| Human placenta | B | 1,9 | (0,78) | 50 |
| Human placenta | B | 50 | (20,6) | 87,5 |
| Rat stomach | B | 2 | (0,82) | 67,2 |
| Rat colon | B | 2 | (0,82) | 53,3 |
| Rat colon | A | 50 | (19,9) | 47,8 |
| Rat colon | C | 50 | (12,8) | 71,3 |
| Rat kidney | B | 2 | (0,82) | 94,1 |
| Rat lung | B | 2 | (0,82) | 85,2 |
| Guinea pig duodenum | B | 2 | (0,82) | 68,3 |
| Guinea pig colon | B | 2 | (0,82) | 58,7 |
| Guinea pig kidney | B | 2 | (0,82) | 53,8 |
| Guinea pig kidney | B | 50 | (20,6) | 65,9 |
| Guinea pig kidney | C | 50 | (12,8) | 45,4 |

As the inhibition expressed as per cent is linear against the logarithm of the concentration, even rather small differences in per cent inhibition are important. The table shows that compound B according to the invention is 50 times as active as the known compound A, which has been the hitherto most active one of earlier known compounds. Even the structurally most simple compound according to the invention, C, is significantly more active than compound A.

The invention also comprises pharmaceutical preparations containing the compounds of the invention in combination with an organic or inorganic inert carrier material suitable for oral, rectal or parenteral application. The pharmaceutical preparations may exist in solid, semi-solid or liquid form, and they may optionally be sterilized and/or contain additional adjuvants. The pharmaceutical preparations may be prepared in a manner well known to every person skilled in the art by mixing the active susbtance with the carrier material and possible additional adjuvanta and converting the mixture obtained into a suitable galenic form. As a dosage guide line a daily dosis of 5—5,000 mg per day may be indicated.

The invention will be illustrated further in the following specific examples, which, however, in no way are intended to restrict the scope of the invention.

Example 1

2-hydroxy-5-[4-[[(2-pyridinyl)-amino]sulfonyl]phenylazo]benzene-acetic acid

12 g sulfapyridine was dissolved with cooling to 0°C in 40 ml water and 12 ml conc. hydrochloric acid. 3.5 g sodium nitrite in 20 ml water was added to the sulfapyridine solution during about 15 minutes with stirring and cooling. The diazonium salt solution was added to 7.6 g 2-hydroxy-benzene-acetic acid and 7 g sodium hydroxide in 50 ml water at 0—10°C. After stirring for 1.5 h the solution was acidified by hydrochloric acid. The precipitating product was filtered off and washed with water. Yield 18.1 g. The product was dissolved in acetic acid and treated with 5 g decolourizing coal. The solution was filtered and evaporated in vacuum. This was repeated once, a pure product being obtained, which during partial melting at about 135°C was converted into another crystal modification of melting point 185—187°C with decomposition.

Example 2

4-[(2-oxo-5(2H)-benzofuranyl-azo]-N-(2-pyridinyl)-benzenesulfonamide

4.0 g of the acid produced according to Example 1 was heated with 65 ml acetic acid, 2 ml acetic anhydride and 0.25 ml conc. sulfuric acid until a clear solution was obtained. 25 ml water was added and the solution was cooled to room temperature, the product crystallizing. After filtration and washing with water 2.1 g of the title compound was obtained, which melted at 231°C with decomposition.

Example 3

2-hydroxy-5-[4-[[(2-pyridinyl)-amino]-sulfonyl]phenylazobenzenepropanoic acid

25.0 g of sulfapyridine was dissolved in 80 ml of water and 24 ml of conc. hydrochloric acid. The solution was kept at 0°C. 7.0 g of sodium nitrite in 40 ml of cold water was added during about 10 min. with stirring and cooling by the addition of 100 g ice in portions.

14.8 g of 3,4-dihydro-(2H)-1-benzopyran-2-one (the lactone of 2-hydroxy-benzene-propanoic acid) was added to 16 g of sodium hydroxide in 200 ml of water. The mixture was heated to 80—90°C until all of the lactone had been hydrolyzed and dissolved. The solution was cooled to about 0°C and the diazonium salt solution quickly added with stirring. After 5 min., the solution was acidified with dilute hydrochloric acid. An oily mass precipitated. After decantation of the aqueous phase and digestion with 200 ml of acetone, the product crystallized and was filtered off. It was dissolved in 500 ml of water and 5 g of sodium hydroxide and carefully precipitated by the addition of acetic acid. The yield was 24.3 g. Melting point 188—190°C.

Example 4

By the application of the procedures given in Examples 1 and 3 with appropriate starting materials the following compounds were obtained after purification by recrystallization from acetic acid, alcohol or ethylene dichloride or by reprecipitation from an alkaline water solution by the addition of a mineral acid, formic or acetic acid:

a) 2-Hydroxy-5-[-4-[[(4,5-dimethyl-2-oxazolyl)amino]sulfonyl]phenylazo]benzenepropanoic acid. Melting Point: 206°C with decomposition.

b) 2-Hydroxy-5-[4-[[(4,6-dimethyl-2-pyrimidinyl)amino]sulfonyl]phenylazo]benzeneacetic acid. Melting point: 180°C with decomposition.

c) 2-Hydroxy-5-phenylazo-benzeneacetic acid. Melting point: 165—167°C.

d) 2-Hydroxy-5-phenylazo-benzenepropanoic acid. Melting point: 147—148°C.

e) 2-Hydroxy-5-(4-methoxy-phenylazo)benzeneacetic acid. Melting point: 211°C with decomposition.

f) 5-(4-Chloro-phenylazo)-2-hydroxy-benzeneacetic acid. Melting point: 193—195°C.

g) 5-(3-Chloro-phenylazo)-2-hydroxy-benzeneacetic acid. Melting point: 166—168°C.

h) 5-(2-Chloro-phenylazo)-2-hydroxy-benzenepropanoic acid. Melting point: 149—151°C.

i) 2-Hydroxy-5-(4-methyl-phenylazo)benzenepropanoic acid. Melting point: 139—141°C.

j) 5-(4-Ethoxycarbonyl-phenylazo)-2-hydroxy-benzeneacetic acid. Melting point: 214—216°C.

k) 5-(3-Chloro-4-methyl-phenylazo)-2-hydroxy-benzenepropanoic acid. Melting point: 127—129°C.

l) 5-(4-Carboxy-phenylazo)-2-hydroxy-benzeneacetic acid. Melting point: 254°C with decomposition.

**Claims**

1. Arylazo compounds of the general formula

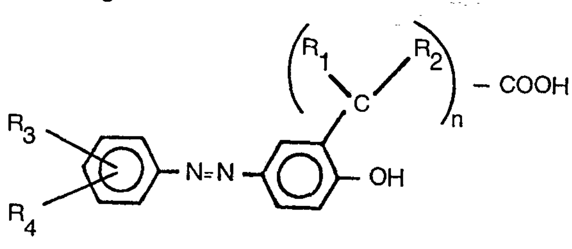

I

wherein $R_1$ and $R_2$ independently of each other represent hydrogen or $C_{1-3}$ alkyl, $R_3$ represents hydrogen, halogen, $C_{1-3}$ alkyl, $C_{1-3}$ alkoxy, carboxy, $C_{1-3}$ alkoxycarbonyl, $C_{1-3}$ alkylcarbonyl or a group X—NH—$SO_2$—, wherein X represents a heterocyclic ring, $R_4$ represents hydrogen, halogen or $C_{1-3}$ alkyl, and n is the integer 1 or 2, the two groups

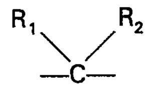

being the same or different when n is 2-, and lactones and salts thereof.

2. Arylazo compounds according to claim 1, characterized in that X is a monocyclic, unsaturated heterocyclic ring, which optionally is substituted by one or more low-alkyl groups or halogen atoms.

3. Arylazo compounds according to claim 1 or 2, characterized in that $R_1$ and $R_2$ are hydrogen.

4. Arylazo compounds according to claim 1, 2 or 3, characterized in that $R_3$ represents hydrogen, halogen, methyl, carboxy, methoxycarbonyl, ethoxycarbonyl, methoxy or X—NH—$SO_2$, wherein X represents a heterocyclic ring, and $R_4$ represents hydrogen.

5. Arylazo compounds according to any one of claims 1 to 4, characterized in that n = 1.

6. Arylazo compounds according to any one of the claims 1 to 5, characterized in that it is 2-hydroxy-5-[4[[(2-pyridinyl)-amino]sulfonyl]phenylazo]benzeneacetic acid.

7. A method of preparing arylazo compounds according to claim 1 of the general formula

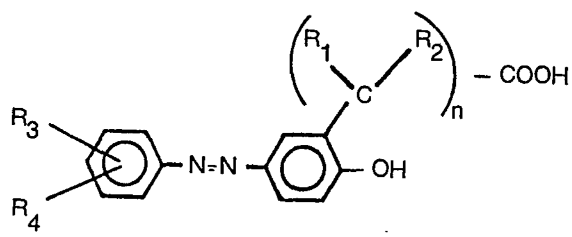

I

wherein $R_1$—$R_4$ and n are as defined in claim 1, characterized by

1) diazotizing a compound of formula

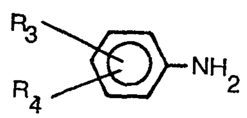

II

wherein $R_3$ and $R_4$ are as defined above,
and in alkaline medium coupling the diazotized product with a compound of formula

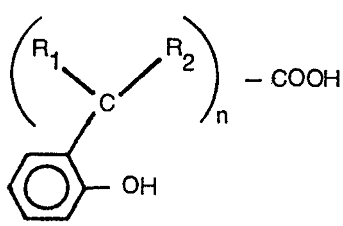

III

wherein $R_1$, $R_2$ and n are as defined above, or

2) reacting a sulfonyl chloride of the formula

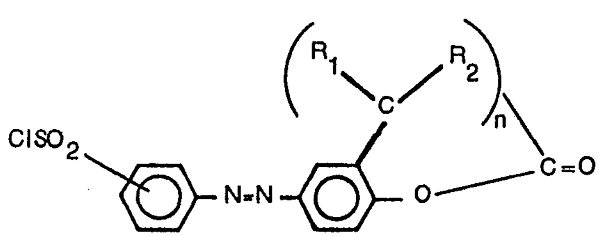

IV

wherein $R_1$, $R_2$ and n are as defined above,
with an amine of the formula

$X—NH_2$ V

wherein X is as defined above, in the presence of a basic condensation agent, and subsequently in a manner known per se optionally converting an open-ring compound of formula I obtained into the corresponding lactone, or vice versa, and optionally converting a resulting compound into a salt.

8. Arylazo compounds according to any one of claims 1 to 6 as pharmaceutically active substances, particularly as inhibitors for prostaglandin synthetizing and inactivating enzymes.

9. A pharmaceutical preparation for treating inflammatory diseases, characterized in that it comprises an arylazo compound according to any one of the claims 1 to 6 in combination with a pharmaceutically acceptable carrier.

10. A male anti-fertility agent, characterized in that it comprises an arylazo compound according to any one of claims 1 to 6 in combination with a pharmaceutically acceptable carrier.

## Revendications

1. Composés arylazo de formule générale

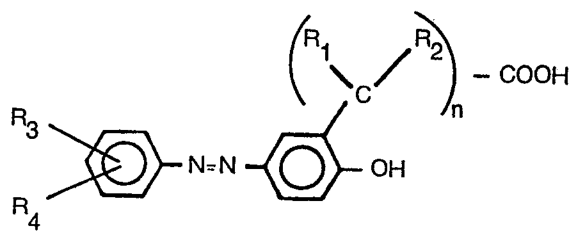

I

dans laquelle $R_1$ et $R_2$, indépendamment l'un de l'autre, représentent l'hydrogène ou un groupe alkyle en $C_{1-3}$, $R_3$ représente l'hydrogène, un halogène, un groupe alkyle en $C_{1-3}$, un groupe alcoxy en $C_{1-3}$, un groupe carboxy, un groupe alcoxycarbonyle en $C_{1-3}$, un groupe alkylcarbonyle en $C_{1-3}$ ou un groupe $X—NH—SO_2—$, dans lequel X représente un hétérocycle, $R_4$ représente l'hydrogène, un halogène ou un groupe alkyle en $C_{1-3}$ et *n* est un nombre entier égal à 1 ou 2, les deux groupes

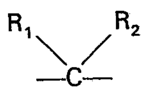

étant identiques ou différents lorsque *n* est 2, ainsi que les lactones et les sels de ces composés.

2. Composés arylazo selon la revendication 1, caractérisés en ce que X est un hétérocycle monocyclique insaturé éventuellement substitué par un ou plusieurs groupes alkyle inférieurs ou atomes d'halogène.

3. Composés arylazo selon la revendication 1 ou 2, caractérisés en ce que $R_1$ et $R_2$ représentent l'hydrogène.

4. Composés arylazo selon la revendication 1, 2 ou 3, caractérisés en ce que $R_3$ représente l'hydrogène, un halogène, un groupe méthyle, carboxy, méthoxycarbonyle, éthoxycarbonyle, methoxy ou $X—NH—SO_2—$, dans laquelle X représente, un hétérocycle et $R_4$ représente l'hydrogène.

5. Composés arylazo selon l'une quelconque des revendications 1 à 4, caractérisés en ce que $n = 1$.

6. Composés arylazo selon l'une quelconque des revendications 1 à 5, caractérisé en ce qu'il s'agit de l'acide hydroxy-2 (pyridinyl-2 amino) sulfonyl-4 phénylazo-5 benzèneacétique.

7. Procédé de préparation de composés arylazo selon la revendication 1, de formule générale

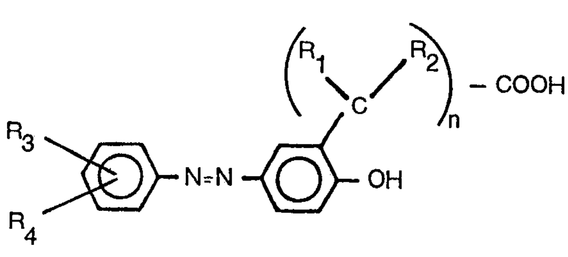

I

dans laquelle les radicaux $R_1$ à $R_4$ et l'indice *n* sont tels que définis dans la revendication 1, caractérisé en ce qu'il consiste à

1) diazoter un composé de formule

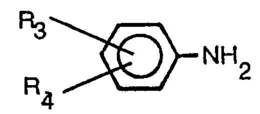

II

dans laquelle $R_3$ et $R_4$ sont tels que définis plus haut, et à coupler, en milieu alcalin, le produit diazoté avec un composé de formule

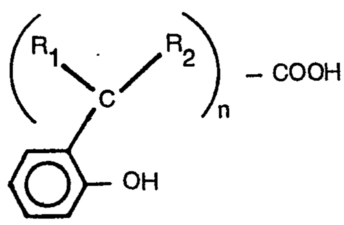

III

dans laquelle $R_1$, $R_2$ et $n$ sont tels que définis ci-dessus, ou

2) à faire réagir un chlorure de sulfonyle de formule

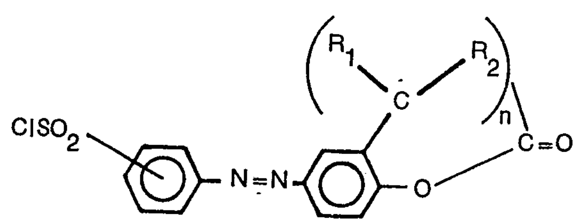

IV

dans laquelle $R_1$, $R_2$ et $n$ sont tels que définis plus haut, avec une amine de formule

$$X—NH_2$$

dans laquelle X est tel que défini ci-dessus, en présence d'un agent de condensation basique, puis, d'une manière connue en soi, à éventuellement transformer un composé à cycle ouvert de formule I obtenu en lactone correspondante, ou vice-versa, et éventuellement à transformer un composé résultant en sel.

8. Composés arylazo selon l'une quelconque des revendications 1 à 6, en tant que substances pharmaceutiquement actives, en particulier comme inhibiteurs d'enzymes de synthèse et d'inactivation de prostaglandines.

9. Préparation pharmaceutique pour le traitement de maladies inflammatoires, caractérisée en ce qu'elle renferme un composé arylazo selon l'une quelconque des revendications 1 à 6, combiné avec un support pharmaceutique convenable.

10. Agent anti-fertilité masculine, caractérisé en ce qu'il renferme un composé arylazo selon l'une quelconque des revendications 1 à 6, combiné avec un support pharmceutique convenable.

**Patentansprüche**

1. Arylazoverbindungen der allgemeinen Formel

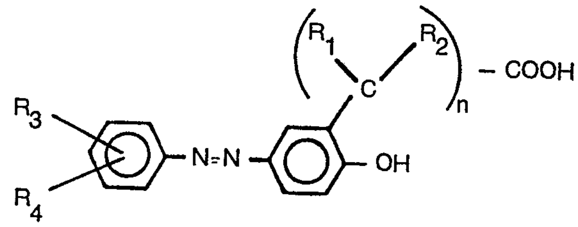

I

worin $R_1$ und $R_2$ unabhängig voneinander für Wasserstoff oder $C_{1-3}$-Alkyl stehen, $R_3$ für Wasserstoff, Halogen, $C_{1-3}$-Alkyl, $C_{1-3}$-Alkoxy, Carboxy, $C_{1-3}$-Alkoxycarbonyl, $C_{1-3}$-Alkylcarbonyl oder eine Gruppe

X—NH—$SO_2$—, worin X einen heterocyclischen Ring bedeutet, steht, $R_4$ für Wasserstoff, Halogen oder $C_{1-3}$-Alkyl steht und n eine ganze Zahl von 1 oder 2 ist, wobei die zwei Gruppen

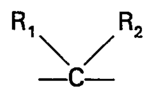

gleich oder verschieden sind, wenn n den Wert 2 hat, sowie die Lactone und Salze davon.

2. Arylazoverbindungen nach Anspruch 1, dadurch gekennzeichnet, daß X ein monocyclischer, ungesättigter heterocyclischer Ring ist, der gegebenenfalls durch ein oder mehrere Niedrigalkylgruppen oder Halogenatome substituiert ist.

3. Arylazoverbindungen nach Anspruch 1 oder 2, dadurch gekennzeichnet, daß $R_1$ und $R_2$ Wasserstoff sind.

4. Arylazoverbindungen nach Anspruch 1, 2 oder 3, dadurch gekennzeichnet, daß $R_3$ für Wasserstoff, Halogen, Methyl, Carboxy, Methoxycarbonyl, Ethoxycarbonyl, Methoxy oder X—NH—$SO_2$, wobei X einen heterocyclischen Ring bedeutet, steht und daß $R_4$ für Wasserstoff steht.

5. Arylazoverbindungen nach einem der Ansprüche 1 bis 4, dadurch gekennzeichnet, daß $n = 1$.

6. Arylazoverbindungen nach einem der Ansprüche 1 bis 5, dadurch gekennzeichnet, daß es sich um 2-Hydroxy-5-[4[[(2-pyridinyl)-amino]sulfonyl]phenylazo]benzolessigsäure handelt.

7. Verfahren zur Herstellung von Arylazoverbindungen nach Anspruch 1 der allgemeinen Formel

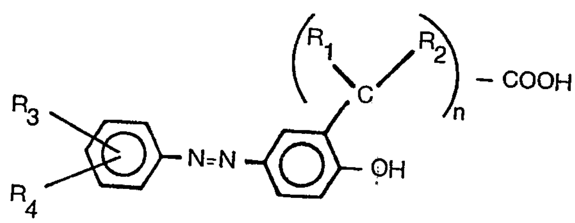

I

worin $R_1$—$R_4$ und n die in Anspruch 1 angegebenen Definitionen haben, dadurch gekennzeichnet, daß man

1) eine Verbindung der Formel

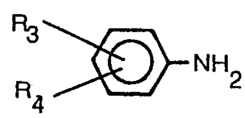

II

worin $R_3$ und $R_4$ die obigen Definitionen haben,diazotisiert und in einem alkalischen Medium das diazotisierte Produkt mit einer Verbindung der Formel

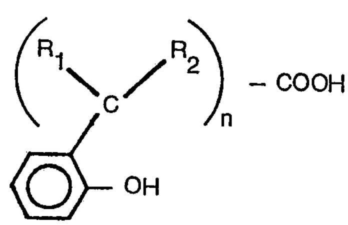

III

worin $R_1$, $R_2$ und n die oben angegebenen Definitionen haben, kuppelt oder daß man

2) ein Sulfonylchlorid der Formel

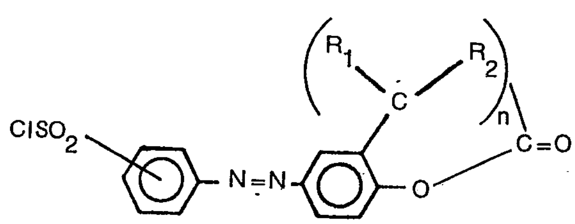

IV

worin $R_1$, $R_2$ und n die oben angegebenen Definitionen haben, mit einem Amin der Formel

X—$NH_2$ V

worin X die obige Definition hat, in Gegenwart eines basischen Kondensationsmittels umsetzt und daß man sodann in an sich bekannter Weise gegebenenfalls eine erhaltene Verbindung der Formel I mit offenem Ring in das entsprechende Lacton oder umgekehrt umwandelt und daß man gegebenenfalls eine resultierende Verbindung in ein Salz umwandelt.

8. Arylazoverbindungen nach einem der Ansprüche 1 bis 6 als pharmazeutisch aktive Substanzen, insbesondere als Inhibitoren für prostaglandinsynthetisierende und -inaktivierende Enzyme.

9. Arzneimittel zur Behandlung von entzündlichen Erkrankungen, dadurch gekennzeichnet, daß es eine Arylazoverbindung nach einem der Ansprüche 1 bis 6 in Kombination mit einem pharmazeutisch annehmbaren Träger enthält.

10. Mittel gegen die Unfruchtbarkeit des Mannes, dadurch gekennzeichnet, daß es eine Arylazoverbindung nach einem der Ansprüche 1 bis 6 in Kombination mit einem pharmazeutisch annehmbaren Träger enthält.